# EUROPEAN PATENT APPLICATION

(11) **EP 1 279 739 A1**
(43) Date of publication of application: **29.01.2003**
(21) Application number: 01870166.4
(22) Date of filing: 26.07.2001
(51) Int. Cl.: C12N 15/86, C07K 14/82, C12N 5/10, A61K 48/00, A61P 35/00

(54) **Recombinant vectors derived from adeno-associated virus expressing TAM67 for gene therapy**

(71) Applicant: VRIJE UNIVERSITEIT BRUSSEL, 1050 Brussel (BE)
(72) Inventor: De Greve, Jacques, B-1700 Dilbeek (BE); Teugels, Erik, B-9255 Huggenhout (BE); Neyns, Bart, B-1730 Asse (Mollem) (BE); Zeinoun, Ziad, B-1090 Brussels (BE); Vermeij, Joanna, B-1080 Brussels (BE)
(74) Representative: Van Malderen, Michel

(57) **Abstract**

The present invention is related to a recombinant adeno associated viral construct comprising at least:
- a first terminal repeat of an Adeno Associated Virus
- a strong heterologous promoter
- an heterologous DNA corresponding to the gene encoding for the *c-jun* mutant protein TAM67, said gene being under the control of said promoter
- a polyadenylation signal, and
- a second terminal repeat of an Adeno Associated Virus

## Description

### Field of the invention

The present invention is related to a new vector having the ability to inhibit cancer cell growth, especially epithelial cancer cell growth, to a host cell containing said vector, to a pharmaceutical composition containing said vector or said cell for the treatment of cancers, especially epithelial cancers, by gene therapy.

### Background of the invention and state of the art

Cancer is one of the most frequent causes of death of both males and females. Many cancers are difficult to treat with current treatment methods. One such example is ovarian cancer. In over eighty percent of cases, ovarian tumors are of the epithelial type and originate from the cellular surface epithelium overlying the ovaries.

In many cases these tumors are extremely difficult to treat, especially in advanced cancer with metastases. Currently available therapies include surgery, radiation therapy, chemotherapy, radioimmunotherapy, cytokine treatment and hyperthermia. All these treatment modalities have important limitations and disadvantages. For example, surgery can only be performed on localised accessible tumors, radiation and chemotherapy are associated with both acute and latent toxicity; radioimmunotherapy and hyperthermia have limited application and effectivity. Moreover, most of these techniques are not discriminating techniques and destroy not only tumor cells but also normal cells, with various side-effects on patients. However, in general, the efficiency of said therapies and their combinations is still unsatisfactory.

More recently, gene therapy has been proposed as a novel approach to treat malignancies. Gene therapy consists of correcting a deficiency or an abnormality or of ensuring the expression of a protein of therapeutic interest, by introducing genetic information into the cell or organ concerned. This genetic information can be introduced either *in vitro* into a cell extracted from the organ, with the modified cell then being introduced into the organism, or directly *in vivo* in the appropriate tissue.

The introduction of a molecule carrying genetic information can be achieved by various methods known in the art. Preferred methods use gene delivery vehicles derived from viruses, including adenoviruses, retroviruses, vaccina viruses and adeno associated viruses. Among these viruses, adeno-associated viruses (AAV) offer certain attractive properties for gene therapy. The wild type AAVs are DNA viruses of relatively small size which integrate, in a stable and site-specific manner, into the genome of the cells they infect. They are able to infect a wide spectrum of cell species and tissue types, without having any effect on cell growth, on cell morphology or on cell differentiation. In particular, they can express a transgene to high levels in epithelial cells. Moreover, so far no human disease has been found to be associated with AAV infection.

Examples of the use of vectors derived from AAVs for transferring genes *in vitro* and *in vivo* have been described in the literature: see in particular, documents WO 91/18088, WO 93/09239, US Patent. No 4,797,368, US Patent No. 5,139,941 and EP 488 528.

However, the genetics of cancer and the molecular mechanisms operating in cells for maintaining the integrity of tissues are so complicated, that the potential ways for treating cancer are numerous and it is impossible to predict *a priori* the efficacy of a potential way of treatment. Different treatment strategies are currently under investigation. For example, one treatment strategy involves the enhancement of immunogenicity of tumor cells *in vivo* by the introduction of cytokine genes. Another treatment involves the introduction of genes that encode enzymes capable of conferring to the tumor cells sensitivity to chemotherapeutic agents. Another strategy involves the delivery of normal tumor suppressor genes and/or inhibitors of activated oncogenes into tumor cells.

Considering the importance of the challenge for obtaining an efficient gene transfer system, efforts to build up new constructs to be tested *in vivo* are always appreciated.

The AP1 complex is a transcriptional complex composed of the Jun and Fos family of DNA binding protooncoproteins. The activation of the AP1 complex has been implicated in numerous biological processes such as cell proliferation, cell differentiation and apoptosis. Also, cellular transformation by many oncogenes results in an elevation of AP1 activity. Study on the expression pattern of the family of jun genes in normal ovarian epithelium and epithelial ovarian cancer identified a high level of c-jun expression in epithelial tumors and cultured ovarian cancer cells that is induced by serum and TPA (Neyns *et al., Oncogene* **12,** 1247-1257 (1996)). In cultured breast cancer cells comparable observations were made (Chen *et al., Mol. Carcinog.* **15**, 215-226 (1996)).

A truncated form of the c-Jun molecule, the TAM 67 molecule, has been shown to inhibit wild-type c-Jun mediated transcription and transformation by quenching endogenous Jun and Fos proteins (Brown *et al., Oncogene* **8,** 877-886 (1993)). This trans-dominant negative c-Jun mutant lacks the c-Jun 5' transactivating domain of c-Jun but possesses a functional c-Jun leucine Zipper and a DNA binding domain. Malignant transformation of rat embryo cells and NIH3T3 fibroblasts by high level of c-jun expression and tumorigenicity of malignant mouse epidermal cells could also be inhibited by TAM 67 (Rapp *et al., Oncogene* **9**, 3493-3498(1994); Domann *et al., Cell Growth Differ.* **5**, 9-16 (1994)). It has also been shown that TAM 67 expression inhibits AP1-transactivating activity and colony formation of MCF human breast cancer cells *in vitro* (Kameda *et al.,* 1993). However, no construct designed for gene therapy purposes and containing the gene encoding for TAM 67 and being able after transfection to inhibit growth of epithelial tumor cells, in particular of epithelial ovarian tumor cells has been proposed until now.

### Aims of the invention

The present invention aims to provide a vector which does not present the drawbacks of the products of the state of the art.

The present invention also aims to provide a new vector having the ability to inhibit epithelial cancer cell growth when transferred therein.

Another aim of the present invention is to provide a pharmaceutical composition containing said vector or said cell for use in genetic therapy.

### Summary of the invention

The present invention is related to a recombinant adeno associated viral construct comprising at least:
- a first terminal repeat of an Adeno Associated Virus
- a strong heterologous promoter
- an heterologous DNA corresponding to the nucleotide sequence encoding for the *c-jun* mutant protein TAM67, said gene being under the control of said promoter
- a polyadenylation signal, and
- a second terminal repeat of an Adeno Associated Virus

With construct is meant a genetic construct, which can be comprised in a plasmid and/or in a recombinant viral particle.

The recombinant adeno associated viral construct may further comprise nucleotide sequences encoding suitable regulatory elements so as to effect expression of the *c-jun* mutant protein TAM67 in a suitable host cell.

The present invention is also related to a host cell genetically transformed by said construct.

Preferably, said host cell is a human tumor cell.

The present invention is also related to a pharmaceutical composition comprising said recombinant adeno associated viral construct or said cell alone or with a pharmaceutically acceptable carrier. Preferably, the recombinant adeno associated viral construct is comprised in a recombinant viral particle.

The present invention is also related to a method for inhibiting the proliferation of cells, comprising at least the step of transferring a sufficient amount of the recombinant adeno associated viral construct according to the invention into said cells.

Preferably, said cells are epithelial cells.

Preferably, said epithelial cells are mammary epithelial cells, ovarian epithelial cells or lung epithelial cells.

Preferably, said cells are cancer cells.

Preferably, said cancer cells are selected from the group consisting of human melanoma cells, human mammary tumor cells, human ovarian tumor cells, lung tumor cells, human sarcoma cells and carcinoma cells.

The present invention is also related to the use of a sufficient amount of the pharmaceutical composition according to the invention for the preparation of a medicament in the treatment and/or the prevention of cancers.

The present invention is also related to a non-human animal, genetically modified by the recombinant adeno associated viral construct according to the invention.

### Brief description of the drawings

The figure 1 represents the pCEP4-TAM67 plasmid.

Figure 2 shows the pTR-UF2 plasmid in which the rAAV cassette was cloned.

Figure 3 represents an outline of the strategy designed to obtain the plasmid encoding the genome of the recAAV-TAM67 virus

Figure 4a shows the effect of rAAV-TAM 67 (MOI 5 Infective Particles (I.P.)/cell) on proliferation of ovarian cancer cells (NIH-OVCAR3) as reflected by the absorbance at 540nm after 3 hours of exposition to Celltiter96®Aqueous One Solution Cell proliferation assay (Promega). Figures 4b and 4c show the same data as figure 4a but presented over time (days)

### Detailed description of the invention

The invention will now be clarified using examples and figures which are not to be considered as limiting to the scope of the invention as defined by the claims.

### Example 1:

### STRATEGY TO OBTAIN RECOMBINANT AAV PARTICLES (recAAV-TAM67)

Conventional methods for obtaining recombinant AAV (recAAV) particles are based on co-transfection of an eucaryotic cell line (mostly 293 cells) and upper-infection with adenovirus. One of the 2 co-transfected plasmids encodes the genome of the recAAV (bordered by a couple of inverted terminal repeats, the only remaining elements of the wtAAV genome), the other plasmid codes for the REP and CAP proteins of the wtAAV necessary for trans-complementation. The REP proteins are responsible for the synthesis of the recAAV genomes, while the CAP proteins are the structural molecules of the viral capsid. In order to realise the production of AAV particles cells have to be "activated", e.g. by upper-infection with adenovirus, otherwise AAV remains under a latent form in the host cell.
- The plasmid coding for the REP and CAP proteins (pIM45; provided by N. Muzyczka)
- The plasmid encoding the recAAV genome (pTR-TAM67) was constructed as described hereafter.

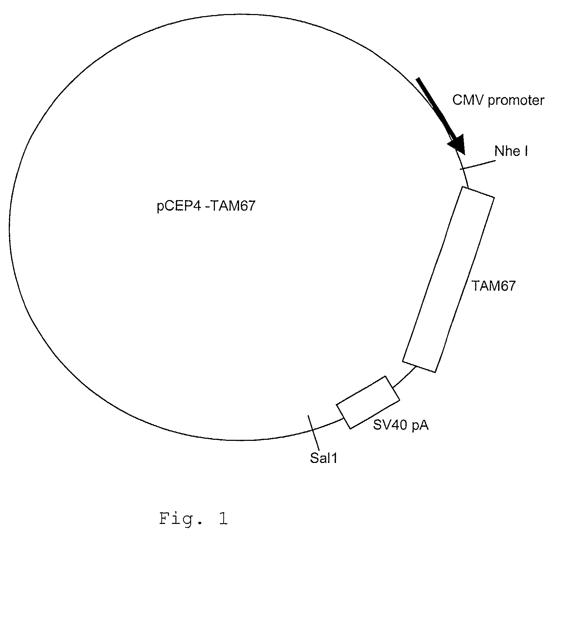

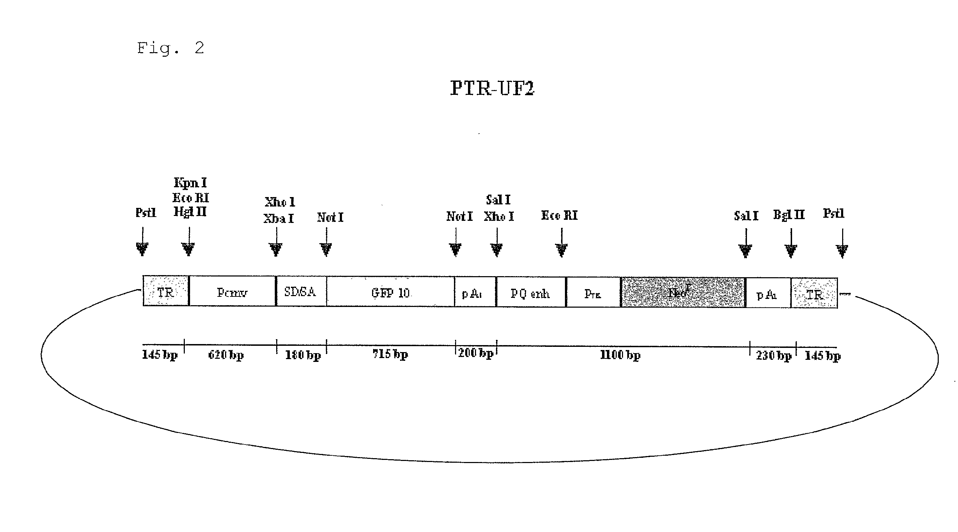

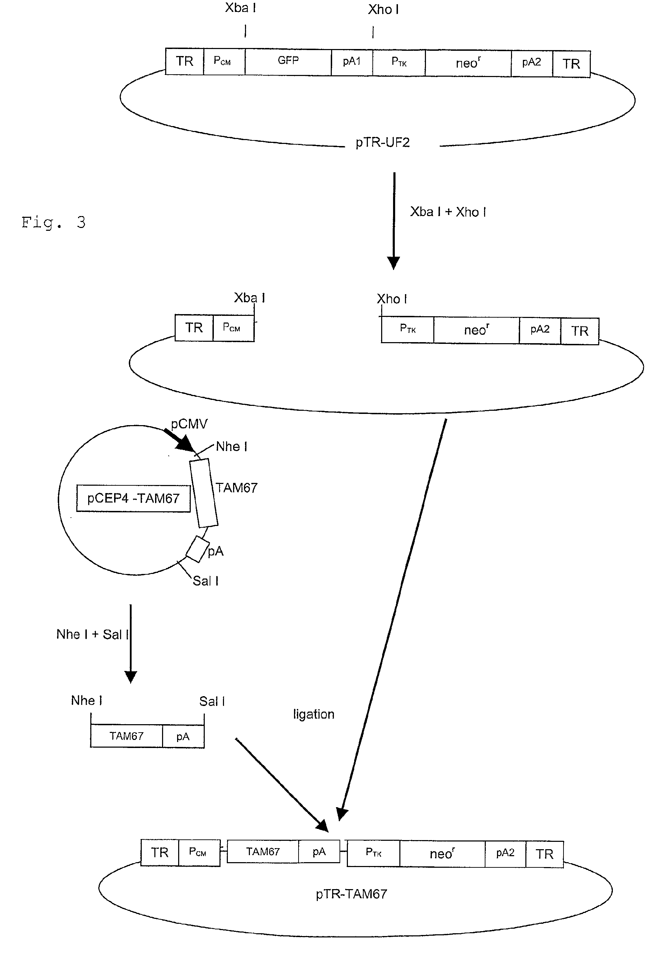

Basically, the TAM67 sequence together with the adjacent CMV promoter was extracted from the pCEP4-TAM67 plasmid (see fig.1, kindly provided by M.J. Birrer) using the Nhe I / Sal I restriction sites. This DNA fragment was inserted into the pTR-UF2 plasmid, previously digested with the Xba I / Xho I restriction enzymes (the sticky ends created by Nhe I are compatible with Xba I ends, idem for Sal I and Xho I). The pTR-UF2 plasmid (see fig.2 , kindly provided by N.Muzyczka, see Zolotukhin S et al., J Virol 70(7):4646-4654, 1996) codes for the genome of a rec AAV designed for expression of a reporter gene (the Green Fluorescent Protein or GFP) and a gene conferring resistance to an antibiotic (neomycin) . In our recAAV plasmid construct (pTR-TAM67), the GFP gene in pTR-UF2 is replaced by a putative therapeutic gene (TAM67, outline of the strategy see fig. 3).

pCEP4-TAM67 is a derivative of the pCEP4 plasmid. (http://www.invitrogen.com/vectordata/index.html) where the blunt ended TAM67 sequence was inserted in the polylinker at the level of the Xho I restriction site between the CMV promoter and the SV40 polyadenylation site. TAM67 is a synthetic cJun mutant created by deletion of amino acids 3-122 using the polymerase chain reaction as described in Alani et al. 1991, Mol. Cell. Biol., 11: 6286-6295.

A deposit has been made according to the Budapest Treaty for the plasmid pTR-TAM67 under deposit number LMBP 4280 at the BCCM/LMBP Plasmid Collection, Laboratorium voor Moleculaire Biologie, Ledeganckstraat 35, B-9000 Gent (Belgium).

### Example 2:

### INFECTION OF OVARIAN CANCER CELLS WITH rAAV-TAM67.

Based on previous experiments with rAAV-GFP (results not shown), in which optimal conditions for transduction of ovarian cancer cells were explored, NIH-OVCAR3 cells were infected with rAAV-TAM67 (at a MOI of 5 infectious particles/cell) with or without addition of Ad5del308ΔpTP (Schaack J, Guo X, Langer SJ. Characterization of a replication-incompetent adenovirus type 5 mutant deleted for the preterminal protein gene. Proc.Natl.Acad.Sci.USA1996; 93: 14686-14691; Maxwell IH, Maxwell F, Schaack J. An adenovirus type 5 mutant with the preterminal protein gene deleted efficiently provides helper functions for the production of recombinant adeno-associated virus. J Virol 1998; 72: 8371-8372) at a MOI of 1 I.P/cell (in the experimental set-up adenovirus is added to obtain maximum expression levels of the recombinant gene). Controls included infection with Ad5del308ΔpTP alone, wtAAV infection (due to our production method the rAAV-TAM67 preparation contains a known amount of wtAAV) and uninfected cells (Mock). On a daily basis (four day period, starting on day 1 after infection) a proliferation assay was performed (Celltiter96®Aqueous One Solution cell proliferation assay, Promega) to assess the effect of TAM67 on these cells.

The absorbance measured in this assay reflects the number of viable cells. The data demonstrate a probable effect of Ad5del308ΔpTP as well as wtAAV by themselves (also reflected in the rAAV-TAM67 without Ad5del308ΔpTP) however the largest reduction in viable cells is obtained when cells are coinfected with rAAV-TAM67 and Ad5del308ΔpTP (provides necessary help for single to double strand conversion of rAAV-TAM67 DNA) indicating an effect of TAM67 on the growth of NIH-OVCAR3. Measurements were not extended beyond four days due to confluence of the Mock control.

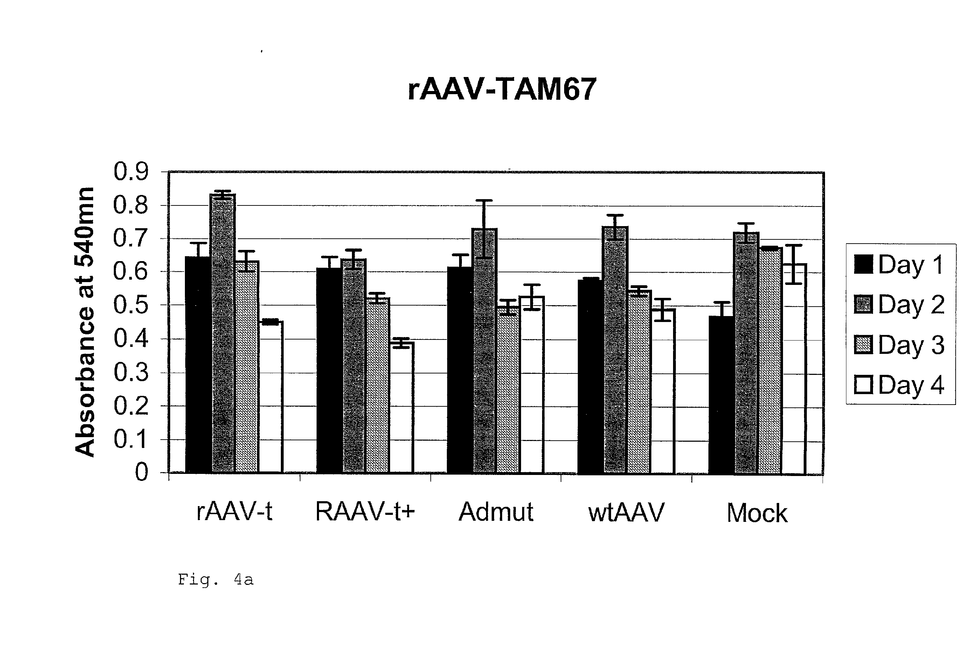

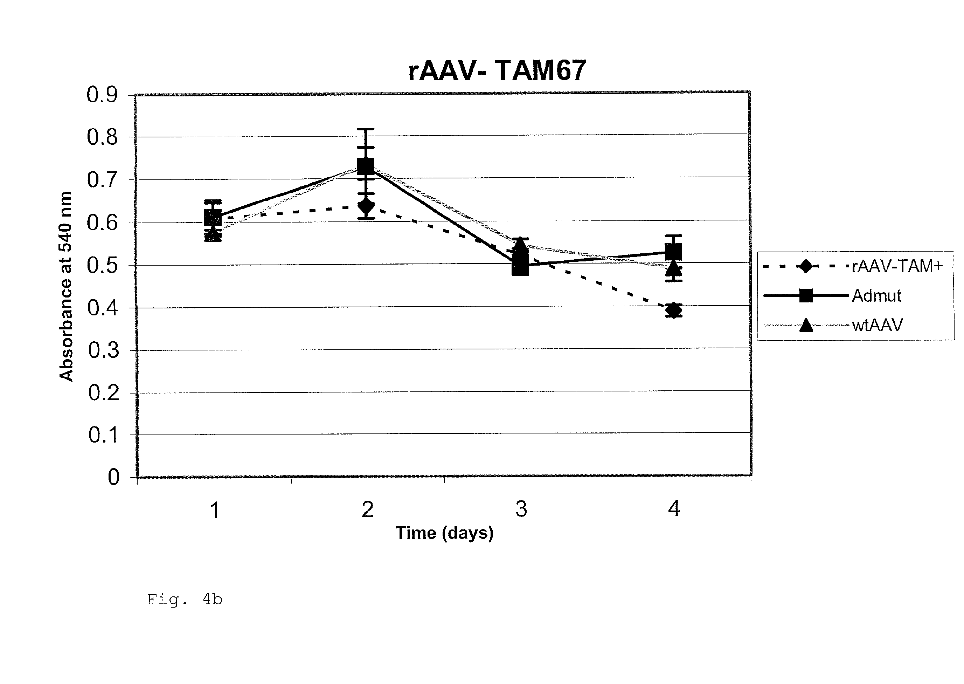

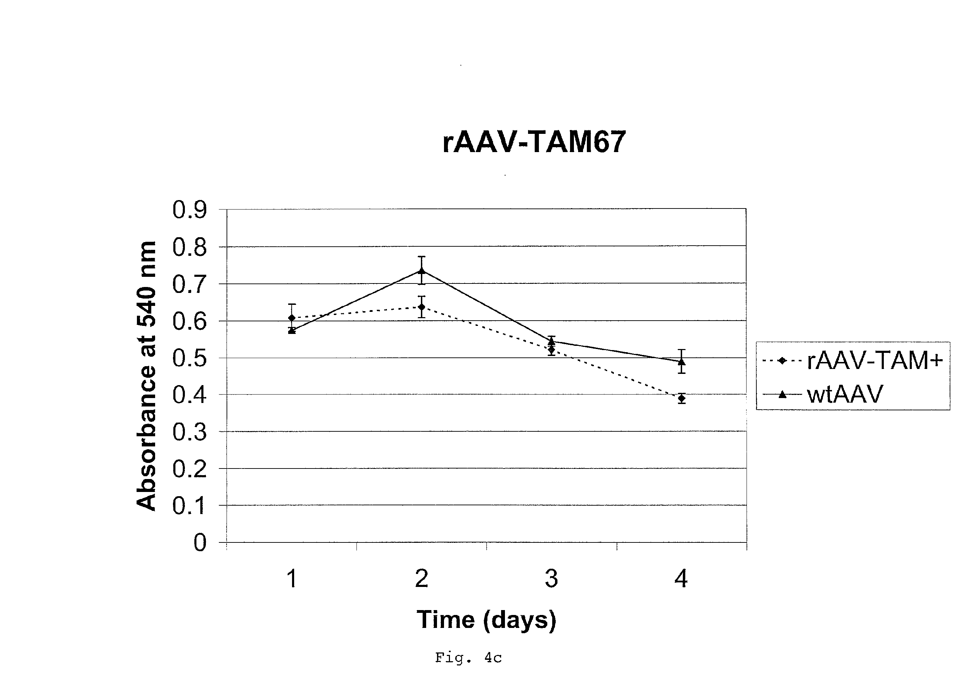

### Legend to Figs 4a-4c

t means TAM67
+ means with addition of Ad5del308ΔpTP (MOI 1 I.P/cell).
Average of three readings (Elisa reader, Biorad). Appropriate controls are included. Controls included are Admut (Ad5del308ΔpTP), wild type AAV (wtAAV) at similar MOI's and uninfected cells (Mock). Observations were not extended beyond four days due to confluence of the Mock control.

## Claims

1. A recombinant adeno associated viral construct comprising at least:
- a first terminal repeat of an Adeno Associated Virus
- a strong heterologous promoter
- an heterologous DNA corresponding to the gene encoding for the *c-jun* mutant protein TAM67, said gene being under the control of said promoter
- a polyadenylation signal, and
- a second terminal repeat of an Adeno Associated Virus

2. The recombinant adeno associated viral construct of claim 1 wherein said construct is comprised in a plasmid and/or a recombinant viral particle.

3. The recombinant adeno associated viral construct of claim 1 or 2 further comprising nucleotide sequences encoding suitable regulatory elements so as to effect expression of the *c-jun* mutant protein TAM67 in a suitable host cell.

4. A host cell genetically transformed by the construct according to claim 1 or 3.

5. The host cell according to claim 4, **characterized in that** said host cell is a human tumor cell.

6. A pharmaceutical composition comprising the recombinant adeno associated viral construct of any of the claims 1 to 3 or the cell according to claim 4 or 5 and a pharmaceutically acceptable carrier.

7. The pharmaceutical composition as in claim 6 wherein the construct is comprised in a plasmid and/or a recombinant viral particle.

8. A method for inhibiting the proliferation of cells, comprising at least the step of transferring a sufficient amount of the recombinant adeno associated viral construct according to any one of the claim 1 to 3 into said cells.

9. The method of claim 8, **characterized in that** said cells are epithelial cells.

10. The method according to claim 9, **characterized in that** said epithelial cells are mammary epithelial cells, ovarian epithelial cells or lung epithelial cells.

11. The method according to claim 9 or 10, **characterized in that** said cells are cancer cells.

12. The method according to claim 11, **characterized in that** said cancer cells are selected from the group consisting of human melanoma cells, human mammary tumor cells, human ovarian tumor cells, lung tumor cells, human sarcoma cells and carcinoma cells.

13. Use of a sufficient amount of the pharmaceutical composition according to claim 6 or 7 for the preparation of a medicament in the treatment and/or the prevention of cancers.

14. Non-human animal, genetically modified by the recombinant adeno associated viral construct according to claim 1 or 3.
